(19) Europäisches Patentamt
European Patent Office
Office européen des brevets

(11) Publication number: **0 452 000 B1**

(12) **EUROPEAN PATENT SPECIFICATION**

(45) Date of publication of patent specification: **18.10.95**

(51) Int. Cl.[6]: **C07C 209/88**, C07C 211/38, C07B 57/00, C07D 493/08, C07C 211/41, A61K 31/00

(21) Application number: **91302753.8**

(22) Date of filing: **28.03.91**

The file contains technical information submitted after the application was filed and not included in this specification

(54) **Preparation of optically active norbornyl amine derivatives.**

(30) Priority: **30.03.90 JP 85443/90**

(43) Date of publication of application:
**16.10.91 Bulletin 91/42**

(45) Publication of the grant of the patent:
**18.10.95 Bulletin 95/42**

(84) Designated Contracting States:
**AT BE CH DE DK ES FR GB GR IT LI LU NL SE**

(56) References cited:
**EP-A- 0 226 346**
**EP-A- 0 226 346**
**FR-A- 2 100 923**

**HOUBEN - WEYL 'methoden der organischen chemie' 1955 , GEORG THIEME VERLAG , VOL IV , PART 2 , STUTTGART**

**PATENT ABSTRACTS OF JAPAN vol. 13, no. 107 (C-576)(3455) 14 March 1989**

**JOURNAL OF AMERICAN CHEMICAL SOCIETY, vol 98, N0 6, pages 8476-81**

(73) Proprietor: **SHIONOGI SEIYAKU KABUSHIKI KAISHA trading under the name of SHIONOGI & CO. LTD.**
**1-8, Doshomachi 3-chome,**
**Chuo-ku**
**Osaka 541 (JP)**

(72) Inventor: **Ohtani, Mitsuaki**
**1342, Takabatake-cho**
**Nara-shi,**
**Nara-ken (JP)**
Inventor: **Takahashi, Hisanori**
**15-8, Terahata 2-chome**
**Kawanishi-shi,**
**Hyogo-ken (JP)**
Inventor: **Watanabe, Fumihiko**
**726-44, Ohaza-shimomaki,**
**Kanmaki-cho**
**Kitakatsuragi-gun,**
**Nara-ken (JP)**
Inventor: **Takayama, Masami, No. 307, Dai-ni Takaida Haitsu**
**22-9, Takaidamotomachi 2-chome**
**Higashiosaka-shi,**
**Osaka-fu (JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 452 000 B1

(74) Representative: **Hardisty, David Robert et al**
**BOULT, WADE & TENNANT**
**27 Furnival Street**
**London EC4A IPO (GB)**

## Description

This invention relates to a process for preparing an optically active compound useful as an intermediate for the preparation of clinically important thromboxane $A_2$ ($TXA_2$) receptor antagonists. More particularly, it relates to a process for resolving a racemic mixture of a compound of formula (I):

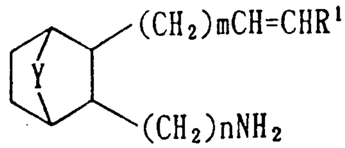

(I)

wherein $R^1$ is hydrogen, lower alkyl or lower alkyl substituted with $-COOR^3$ at the terminal methyl residue; $R^3$ is lower alkyl; Y is oxygen or methylene; m and n are independently 0 or 1 with a chiral acid. As can be seen from the structure, the compound of formula (I) has stereoisomers and their optical isomers, owing to the presence of substituents at 2- and 3-positions of cyclohexane ring. Accordingly, the compound (I) represents all of them. Because typical compounds of formula (I) where Y is methylene are known as "norbornane derivatives", the compounds of formula (I) are hereinafter referred to as norbornyl amine derivatives irrespective of what Y represents.

A stereoisomer of above compound (I) which is shown by formula (I'):

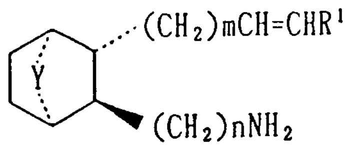

(I')

wherein, $R^1$, Y, m and n are as defined above is useful as an intermediate for the production of a class of optically active 1,4-bridged-cyclohexane carboxylic acid derivatives, which act as $TXA_2$ receptor antagonists, of formula (IV):

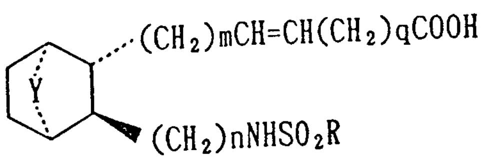

(I V)

wherein, R is phenyl or phenyl substituted with hydroxy, lower alkoxy, halogen, or lower alkyl; Y is oxygen, methylene, substituted methylene; m is 0 or 1; n is 0, 1 or 2; q is 3 or 4 with the proviso that when m is 1, n is 0 or 1.

$TXA_2$ is a member of prostanoids which are biologically active substances synthesized enzymatically from arachidonic acid in various animal tissues. $TXA_2$ has been proved to exhibit many significant biological activities, such as aggregation of platelets and contraction of smooth muscle of various organs. Therefore, $TXA_2$ receptor antagonists have been expected to be therapeutically and prophylactically effective on $TXA_2$-associated diseases. Such diseases include myocardial infraction, pulmonary embolism, thrombosis, encyopyelitis, renal dysfunction, cerebral infraction, asthma caused by bronchoconstriction, and the like. It may be also useful to prevent the vascular contraction after a subarchnoidal bleeding, $TXA_2$ shocks after the artery reperfuse of circulation systems or digestive organs, shocks caused by bleeding, septicemia, wounds, cardiac dysfunction, endotoxin, acute pancreatitis, burns, or the like. It may be effective for the prevention of thrombocytopenia during extracorporeal circulation.

In view of the above, the present inventors had made extensive study and found a class of 1,4-bridged cyclohexane carboxylic acids having antagonistic activities against $TXA_2$ [JP-A-63139161]. Further investigations proved that certain optical isomers thereof, which are generally shown by the above formula (IV), are superior to the racemic mixtures. A typical compound named (+)-S-145 shown by above formula (IV) where R is phenyl, Y is methylene, m is 1, n is 0, and q is 3 has been proved to be a highly effective $TXA_2$

receptor antagonist and clinically useful.

Therefore, it is desirable to produce preferentially optical isomers of formula (IV) to improve the therapeutical effects of anti-$TXA_2$ treatments on previously described diseases. The optically active carboxylic acids (IV) have been mainly obtained by resolving a racemic mixture thereof using conventional methods such as chromatography. However, such resolution method was not effective because the carboxyl moiety is too far removed from the chiral center in the compound (IV) and was not applicable to a large scale production of said optical isomer of formula (IV).

Although asymmetric synthesis can be used, there still remains some problems in yield, cost, and the like.

To overcome these drawbacks, it was suggested to use an optically active intermediate in the early stage of the total process for producing the carboxylic acid (IV). For example, a method for producing the exemplified carboxylic acid ((+)-S-145) has been provided. Thus, (+)-S-145 has been prepared by a process where an optically active sulfonamide intermediate was used. The sulfonamide compound was prepared according to the following reaction scheme.

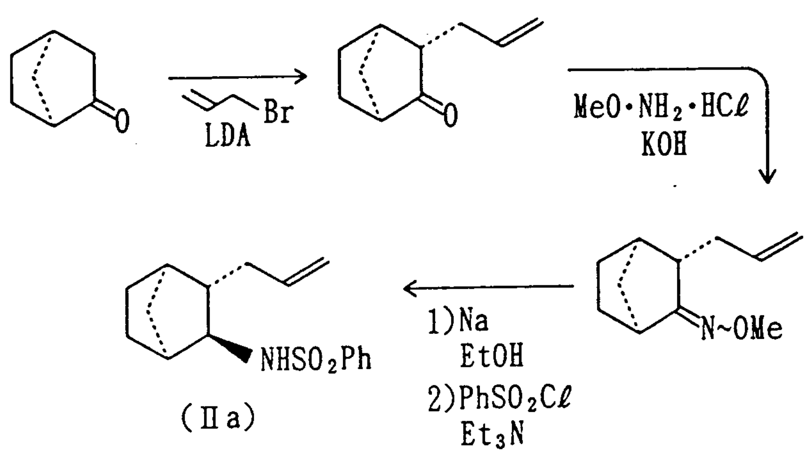

(IIa)

The optically active starting material, norcamphor, was prepared according to the procedures described by J.B.Jones (JACS, 98:8476 (1976)). The poor yield of norcamphar, however, lowered the overall yield of the sulfonamide, resulting in insufficient yield of (+) -S-145. Therefore, it has been highly desired to obtain an optically active intermediate useful to improve the overall yield of the optically active carboxylic acid (IV) and a method for preparing said intermediate.

In "Methoden der Organischen Chemie", Vol. IV, Part 2, 1955, at pages 513 v. 519 and in FR-A-2100923 there are described processes for optical resolution of racemic mixtures, respectively of phenylethylamine and bornane-amine using chiral acids.

The inventors have found that a racemic mixture of norbornyl amine of formula (I):

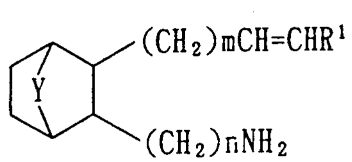
( I )

wherein R, Y, m and n are as defined above, when treated with a certain chiral acid, forms diastereomeric salts consisting of diastereoisomers which differ from each other in physicochemical property, e.g., solubility in a selected solvent, whereby the salt of a desired optical isomer is easily separated from the other.

Thus, the present invention provides a method for resolving a racemic mixture of norbornyl amine of formula (I) by treating said racemic mixture with a chiral acid in an appropriate solvent. An appropriate solvent is selected from various solvents in which either isomer of a chiral salt formed can hardly or never dissolve so that a resultant diastereomeric salt can be separated conveniently by, for example, filtration. Preferably, a solvent in which the salt of desired isomer of compound (I) hardly or never dissolve is selected for purposes of convenience of recovery and purification. Once the desired diastereomeric salt is

separated as a solid, optically active amine of formula (I) can be liberated conventionally by treating it with a base such as metal hydroxide (e.g., lithium hydroxide, sodium hydroxide, potassium hydroxide triethyl amine, and DBN).

Although the present invention is mainly described in the specification in respect of the preparation of a trans isomer (I') of norbornyl amine, the skilled in the art will appreciate that the corresponding cis isomer can be prepared by a procedure similar to that for the trans isomer. For the purpose of the invention, the optical isomer of amine (I) so obtained is then employed for the next step of the total production of carboxylic acid (IV).

Alternatively, the chiral salt obtained as described above can be used for the next step under a basic condition, without liberation of said amine.

Thus, an optically active amine of formula (I) or a chiral salt thereof is reacted with a substituted sulfonyl halide of formula (III):

$RSO_2X$ (III)

wherein R is as defined above; X is halogen to obtain an optically active norbornane-sulfonamide derivative of formula (II):

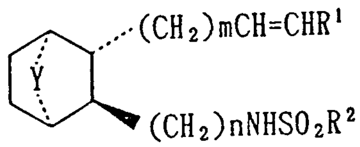

(II)

wherein $R^1$, $R^2$, Y, m, and n are as defined above, which is also useful as an intermediate for the efficient production of carboxylic acid of formula (IV) as previously explained.

Therefore, in another embodiment, the present invention provides a process for preparing an optically active norbornyl amine of formula (I') , which comprises the steps:

(a) treating a racemic mixture of a compound of formula (I) as defined above with a chiral acid in a solvent to form a chiral salt, where the solvent is selected from those in which the diastereomeric salt of said isomer (I') cannot dissolve.

(b) separating precipitates containing said chiral salt formed in step (a); and

(c) recovering the optically active amine of formula (I') as defined above by treating the precipitate obtained in step (b) with a base.

For the purpose of the present invention, as disclosed and claimed herein, the following terms are defined as below.

The term "lower alkyl" refers to a straight or branched saturated hydrocarbon radical having one to eight carbon atoms, including methyl, ethyl, n-propyl, isopropyl, butyl, isobutyl, sec-butyl, tert-butyl, pentyl, isopentyl, neopentyl, 1-methylbutyl, 1,2-dimetylbutyl, hexyl, heptyl, octyl, and the like.

In the definition of "substituted sulfonyl halide", the term "halogen" refers to chlorine, bromine, and the like. Examples of substituents for the "substituted sulfonyl halide" are phenyl or phenyl substituted with hydroxy; halogen such as chlorine, bromine, and the like; lower alkyl such as methyl, ethyl, and the like; and lower alkoxy such as methoxy, ethoxy, and the like. Examples of substituted sulfonyl halide are benzenesulfonyl chloride, methoxybenzenesulfonyl chloride, hydroxybenzenesulfonyl chloride, toluenesulfonyl chloride, bromobenzenesulfonyl bromide, and the like.

Although all compounds of formula (I) are suitable for the method of the present invention, certain classes of compounds are especially preferred for the purpose of the invention. Preferred norbornyl amines are those compounds of formula (I) wherein $R^1$ is hydrogen or propyl having $COOR^3$ at the 3-position thereof; $R^3$ is lower alkyl; Y is methylene or oxygen; and m is 0 or 1 and n is 0 or 1. Particularly preferred norbornyl amine is a compound of formula (I) wherein $R^1$ is hydrogen; Y is methylene; m is 1; and n is 0.

Preferred optically active sulfonamide derivatives of formula (II) are those obtainable from the preferred amines as mentioned above. Specifically, they are shown by formula (II) wherein $R^1$ is hydrogen or propyl having $COOR^3$ at the 3 position thereof; $R^2$ is phenyl, p-tolyl, 4bromophenyl. A compound shown by formula (II) wherein $R^1$ is hydrogen, $R^2$ is phenyl; Y is methylene; and m is 1 and n is 0 is novel and particularly useful as an intermediate for the production of above mentioned (+)-S-145.

The chiral acid to be used in the present invention is acid, N-acetyl-L-glutamic acid or, N-tert-butoxycarbonyl-L-methionine. The resolving agent is generally used in the ratio of 0.5 to 1 mole per 1 mole

of compound (I) as a racemic mixture.

The racemic mixture of norbornyl amine derivative (I), the starting material of the present invention, can be prepared according to various procedures well-known in the field of organic chemistry from a number of compounds. The procedures can be found in literatures including the JP-A-63139161 and JP-A-63284158.

Although the method for resolving a racemic mixture of amine by forming diastereoisomeric salt with a chiral acid has been known, no reports have shown that a racemic mixture of norbornyl amine of formula (I) having alkyl or alkylene side chains at the 2-position of the cyclohexane ring was successfully resolved so far.

According to the present invention, the resolution is accomplished by treating a racemic mixture of compound (I) with a selected chiral acid in a solvent, for example, an alcoholic solvent such as methanol, ethanol, or propanol and allowing the mixture to react at -20 to 100 °C, preferably 0 to 80°C for 1 minute to 60 minutes, preferably 1 minute to 30 minutes. When the reaction mixture is allowed to stand at 0 °C to 40 °C for 1 hour to 24 hours, either isomer of diastereomeric salt precipitates.

Examples of preferred solvents are organic solvents such as alcoholic solvents including methanol, ethanol, and the like.

Purification can be conducted by, for example, recrystallization. The precipitated chiral salt is separated by, for example, filtration and an optically active norbornyl amine is recovered by treating the precipitate conventionally, for example, with a base such as sodium hydroxide, potassium hydroxide, or triethylamine. When the free amine is reacted with a sulfonyl halide of formula (III), an optically active sulfonamide of formula (II) is obtained in high optical yield.

Alternatively, the above salt can be reacted with sulfonyl halide (III) in the presence of a base such as sodium hydroxide, potassium hydroxide, or triethylamine in a solvent such as an alcoholic solvent, water, ethylacetate, and the like, or a mixture thereof to obtain optically active sulfonamide derivative (II) in high optical yield.

Both of the above methods for the production of sulfonamide (II) have advantages over conventional methods including the previouly described one where optically active norcamphor is used as a starting material.

As previously mentioned, the optically active sulfonamide derivative (II) is especially useful as an intermediate for the mass production of the final product, an optically active carboxylic acid derivative of formula (IV).

Thus, according to the present invention, a method for the efficient production of an optically active intermediate useful in the synthetic process for carboxylic acid of formula (IV) is provided.

The following example is set forth to further describe the invention but in no way meant to be construed as limiting the scope thereof.

Example 1

Optical Resolution of Racemic Mixture of Norbornyl Amine using N-Boc-L-methionine

To a solution of 2.49g (10 mM) of N-Boc-L-methionine in 10ml of ethanol is added 3.02g (20 mM) of racemic mixture of amine (Ia) at room temperature and the mixture is allowed to stand for 1 hour. The resultant precipitate is collected by filtration and dried to obtain 2.26g of crude N-Boc-L-methionine salt of amine (Ia). Recrystallization from ethanol-ethyl acetate (I:I) gives pure salt. Yield = 1.27g (15.9%).

The resultant chiral salt is then sulfonylated as described in the following procedure to obtain an optically active sulfonamide (IIa).

To a suspension of 600 mg (1.99 mM) of the chiral salt in 20 ml of ethyl acetate are added 6.6ml (1.99 mM x 3.3) of 1N KOH and 2.79ml (1.99 mM x 1.1) of phenylsulfonyl chloride at 0°C and the mixture is stirred for another 15 minutes at the same temperature. The reaction mixture is washed with 2N HC1, dried over sodium sulfate, and concentrated under reduced pressure. The residue is purified by silica gel column chromatography using 20 g of $SiO_2$ with a mixture of n-hexane and ethyl acetate (9:1) as eluent to obtain optically active sulfonamide (IIa). Yield = 96.0%ee.

Example 2

Optical Resolution of Racemic Mixture of Norbonyl Amine using N-acetyl-L-glutamic acid

To a solution of 3.0g (16 mM) of N-acetyle-L-glutamic acid in 120 ml of methanol is added 3.02g (20 mM) of racemic mixture of amine (Ia) at 30°C. The reaction mixture is concentrated under reduced pressure

to remove methanol. Ethyl acetate is added to the residue and resulting crystals are collected by filtration. Recrystallization from ethanol-ethyl acetate (5:7) gives N-acetyl-glutamic acid salt of amine (Ia). Yield = 1.40 g (20.6%).

The resultant chiral salt is then sulfonylated in the same manner as described in Example 1 to obtain an optically active sulfonamide (IIa) (91.7%ee).

The racemic mixture of amine (Ia) was resolved using various optically active acids and the resultant salts were converted into sulfonamides of formula (IIa) in the same manner as Example 1 described above. The results are summarized in the following Table 1.

Table 1

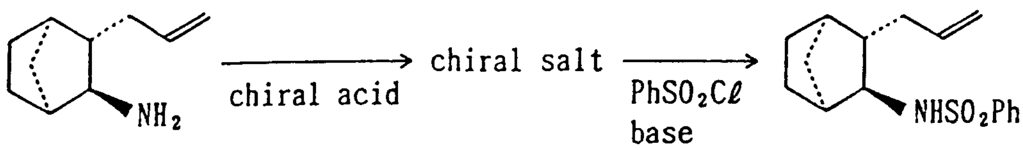


(±)-(Ia)

(IIa)

| resolving agent | recrystallization solvent | Yield of chiral salt of (Ia) | Sulfonamide (IIa) optical rotation | Sulfonamide (IIa) yield (ee) |
|---|---|---|---|---|
| N-Boc-L-methionine | EtOH:AcOEt (1:1) | 15.9% | more than +22.2° | 96.0% |
| N-acetyl-L-glutamic acid | EtOH:AcOEt (5:7) | 20.6% | +22.3° | 91.7% |

* : The yield of salt is based on the starting material, amine (Ia) as a racemic mixture.

The following experiment was conducted to demonstrate the efficiency of the method of the invention in comparison with that of the currently used one.

Experiment

Preparation of Sulfonamide using Optically Active Norcamphor

A. Preparation of (+)-norcamphor and (-)-norcamphor from (±)-exo-2-norbornal

(+)- and (-)-Norcamphor were prepared according to the procedure of J. B. Jones, JACS, 98: 8476-(1976).

(+)-norcamphor:
Yield: 10% (on the basis of (±)-exo-2-norbornal)
$[\alpha]_D$ : +28.6° ( value in the literature = +29.1°) (-)-norcamphor:
Yield: 4.7% (on the basis of (±-exo-2-norbornal)
$[\alpha]_D$ : -28.6° ( value in the literature = -28.7°)

B. Preparation of Phenyl Sulfonylamino Derivative (IIa)

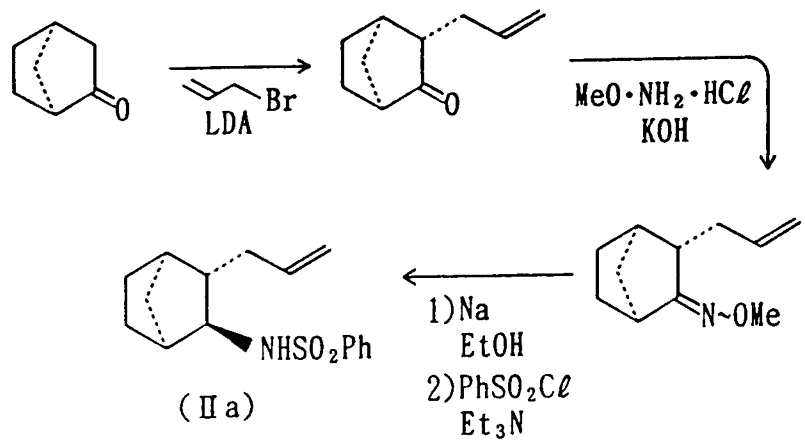


(IIa)

Norcamphor (99%ee) obtained in A. above is treated with allyl bromide and then LDA (1.05 eq.) to introduce an allylic side chain to the bicyclo[2.2.1]heptane ring. The product is converted into oxime by treating with methoxyamine hydrochloride and powdered potassium hydroxide in methanol. The oxime is then reduced with sodium in ethanol to obtain the amino compound, which is then sulfonyated with phenyl sulfonyl chloride to obtain the optically active allyl sulfonamide (IIa). Yield = 92%; M.p. = 98 - 100 °C.

$[\alpha]_D$ = +24.3° ± 0.6° ($CHCl_3$, C = 1.006, 24°C)

| Elemenatal analysis (as $C_{16}H_{21}NO_2S$) | | | | |
|---|---|---|---|---|
| Calculated (%) | C: 65.94; | H: 7.26; | N: 4.81; | S: 11.00 |
| Found (%) | C: 65.99; | H: 7.29; | N: 4.84; | S: 11.04 |

IR($\nu$max)/$CHCl_3$: 3390, 3285, 1326, 1159, 1096 $cm^{-1}$

As can be seen from the results of Examples and Experiment, there is provided a simple and improved method for the preparation of an optically active intermediate useful for the production of carboxylic acid of formula (IV) and an optically active norbornyl amine which is a useful intermediate in the synthetic process for said carboxylic acid.

## Claims

**1.** A process for optical resolution of a racemic mixture of norbornyl amine of formula (I):

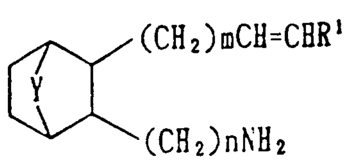

(I)

wherein $R^1$ is hydrogen, $C_1$-$C_8$ lower alkyl or $C_1$-$C_8$ lower alkyl substituted with -$COOR^3$ at the terminal methyl residue; $R^3$ is $C_1$-$C_8$ lower alkyl; Y is oxygen or methylene; m and n are independently 0 or 1, which comprises treating said racemic mixture with a chiral acid selected from the group consisting of N-acetyl-L-glutamic acid and N-tert-butoxycarbonyl-L-methionine in an alcoholic solvent to form a diastereomeric salt.

**2.** A process as claimed in claim 1 wherein the alcoholic solvent is methanol, ethanol, or propanol.

3. A process as claimed in claim 1 or claim 2 wherein a chiral salt formed is separated by filtration and treated with a base to obtain an optically active norbornyl.

4. A process as claimed in any one of claims 1 to 3 wherein the norbornyl amine is

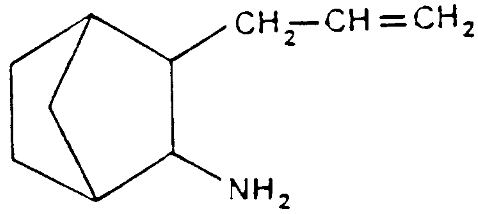

whereby there is obtained a chiral salt of norbornyl amine of the formula:

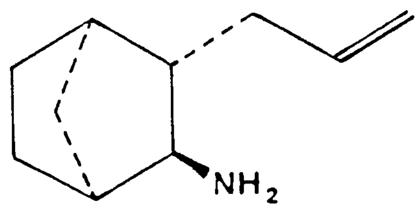

5. A process as claimed in any one of claims 1 to 3, wherein a diastereomeric salt of a norbornyl amine of the formula (I'):

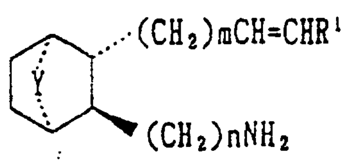

(I')

is formed.

## Patentansprüche

1. Verfahren zur optischen Trennung einer racemischen Mischung von Norbornylamin der Formel (I):

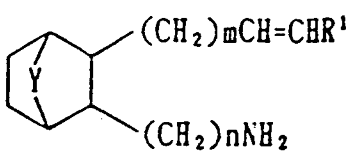

(I),

worin $R^1$ Wasserstoff, $C_1$-$C_8$-nied.Alkyl oder $C_1$-$C_8$-nied.Alkyl substituiert mit -$COOR^3$ am terminalen Methylrest bedeutet; $R^3$ $C_1$-$C_8$-nied.Alkyl darstellt; Y Sauerstoff oder Methylen ist; m und n unabhängig 0 oder 1 sind; welches das Behandeln der racemischen Mischung mit einer chiralen Säure, ausgewählt aus der Gruppe bestehend aus N-Acetyl-L-glutaminsäure und N-tert.Butoxycarbonyl-L-methionin, in einem alkoholischen Lösungsmittel umfaßt, wobei ein diastereomeres Salz gebildet wird.

2. Verfahren nach Anspruch 1, bei welchem das alkoholische Lösungsmittel Methanol, Ethanol oder Propanol ist.

3. Verfahren nach Anspruch 1 oder 2, bei welchem ein gebildetes chirales Salz durch Filtration getrennt und mit einer Base behandelt wird, wobei ein optisch aktives Norbornylamin erhalten wird.

4. Verfahren nach einem der Ansprüche 1 bis 3, bei welchem das Norbornylamin

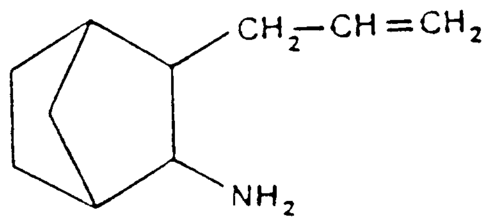

ist, wodurch ein chirales Salz von Norbornylamin der Formel:

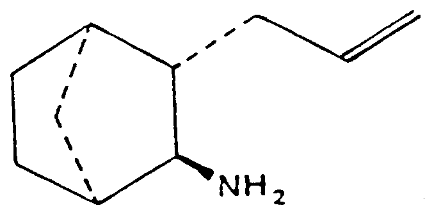

erhalten wird.

5. Verfahren nach einem der Ansprüche 1 bis 3, bei welchem ein diastereomeres Salz eines Norbornylamins der Formel (I')

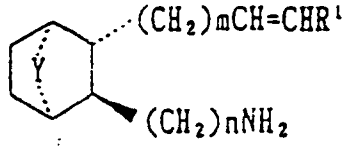

(I')

gebildet wird.

**Revendications**

1. Procédé de résolution optique d'un mélange racémique de norbornylamine de formule (I):

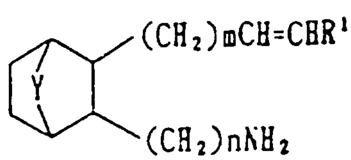

(I)

dans laquelle $R^1$ est l'hydrogène, un alcoyle inférieur en $C_1$-$C_8$ ou un alcoyle inférieur en $C_1$-$C_8$ substitué par -$COOR^3$ au radical méthyle terminal; $R^3$ est un alcoyle inférieur en $C_1$-$C_8$; Y est l'oxygène ou le méthylène; m et n sont, indépendamment l'un de l'autre, 0 ou 1, selon lequel on traite ledit mélange racémique par un acide chiral choisi dans le groupe consistant en l'acide N-acétyl-L-glutamique et la N-tert.butoxycarbonyl-L-méthionine dans un solvant alcoolique, pour former un sel diastéréo-isomère.

2. Procédé selon la revendication 1, dans lequel le solvant alcoolique est le méthanol, l'éthanol ou le propanol.

3. Procédé selon la revendication 1 ou 2, dans lequel le sel chiral formé est séparé par filtration et traité par une base, pour obtenir un composé norbornylamine optiquement actif.

4. Procédé selon l une quelconque des revendications 1 à 3, dans lequel la norbornylamine est

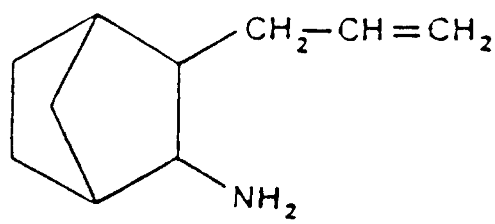

par lequel on obtient un sel chiral de la norbornylamine de formule:

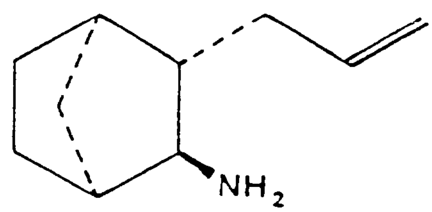

5. Procédé selon l'une quelconque des revendications 1 à 3, dans lequel il se forme un sel diastéréoisomère d'une norbornylamine de formule (I'):

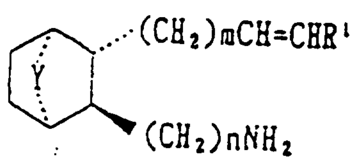

(I')